# EUROPEAN PATENT SPECIFICATION

(11) **EP 1 546 095 B1**
(45) Date of publication and mention of the grant of the patent: **29.03.2006**
(21) Application number: 03751617.6
(22) Date of filing: 30.09.2003
(51) Int. Cl.: C07D 201/08

(54) **PROCESS FOR THE PREPARATION OF e-CAPROLACTAM FROM A MIXTURE COMPRISING 6-AMINOCAPROAMIDE AND/OR OLIGOMERS**
VERFAHREN ZUR HERSTELLUNG VON EPSILON-CAPROLACTAM VON EINER MISCHUNG ENTHALTEND 6-AMINOCAPROAMID UND/ODER OLIGOMERE
PROCEDE DE PREPARATION DE e-CAPROLACTAME A PARTIR D'UN MELANGE COMPRENANT DU 6-AMINOCAPROAMIDE ET/OU DES OLIGOMERES

(30) Priority: 01.10.2002 EP 02079109
(43) Date of publication of application: 29.06.2005
(73) Proprietor: DSM IP Assets B.V., 6411 TE Heerlen (NL)
(72) Inventor: GUIT, Rudolf, Philippus, Maria, NL-6228 GP Maastricht (NL)
(74) Representative: Verhaegen, Ilse Maria M.
(86) International application number: PCT/NL2003/000670
(87) International publication number: WO 2004/031146

(56) References cited:
- WO-A-98/37063
- DE-A- 10 021 192
- US-A- 5 495 016

## Description

The invention relates to a process for the preparation of ε-caprolactam from a mixture comprising 6-aminocaproamide and/or oligomers thereof.

Such a process is described in WO-A-9837063. Example Vill of this publication describes the preparation of caprolactam from a mixture comprising 6-aminocaproic acid, 6-aminocaproamide and oligomers thereof at a pressure of 1.2 MPa and a temperature of 300°C which mixture is obtained by distilling off most of the water from an aqueous reductive amination mixture. In this process the ammonia chemically bound in 6-aminocaproamide and in oligomers thereof (with amide end-groups) releases at the cyclisation conditions. The obtained caprolactam is present in a gaseous product stream also comprising steam, the released ammonia, lights (compounds having a boiling point lower than caprolactam) and heavies (compounds having a boiling point higher than caprolactam). It has been found that the gaseous product stream also comprises carbon dioxide. WO-A-9837063 does not provide a method for separating and recycling the released ammonia to a process in which ammonia is used as for example a reactant. In a continuous process, it would be very favorable for economic reasons if such released ammonia could be separated in a simple way, recycled to and re-used in a process in which ammonia is for example used as reactant, for example in a reductive amination usually catalysed with a hydrogenation catalyst. When the released ammonia is separated from the product stream in the usual way with distillation the carbon dioxide ends up in the released ammonia. Separating ammonia and carbon dioxide is very difficult. In case the thus separated ammonia comprising carbon dioxide would be re-used and recycled to for example a reductive amination, the carbon dioxide would build up in the ammonia recycle of the reductive amination reaction. This is disadvantageous because the total pressure should be elevated in order to be able to operate at constant hydrogen pressure. In order to avoid this, the ammonia recycle of the reductive amination reaction must be at least regularly purged. Purging the ammonia recycle is disadvantageous as this result in loss of valuable products like ammonia.

The object of the present invention is to provide a process for the preparation of caprolactam from 6-aminocaproamide and/or oligomers thereof in which the released ammonia contains at least a strongly reduced amount of carbon dioxide.

This object is achieved in that in a first step 6-aminocaproamide and/or oligomers thereof is converted at a temperature of between 180 and 250°C into ammonia and 6-aminocaproic acid and/or oligomers thereof, with a separate or simultaneous removal of the ammonia, and in a subsequent step the 6-aminocaproic acid and/or oligomers thereof are converted into caprolactam at a temperature of between 250 and 400°C.

It has been found that the ammonia chemically bound in 6-aminocaproamide and/or oligomers thereof which is released in the first step of the process of the invention can be recycled to for example a catalysed reductive amination while the necessity of purging the ammonia recycle of the reductive amination reaction is considerably reduced or even becomes superfluous.

An advantage of the process of the invention is that the amount of degradation compounds which are slightly steam-volatile to a certain extent like for example pentanoic acid, hexanoic acid, hexenoic acid, pentane amide and hexane amide which end up in the released ammonia is considerably reduced compared to the process as described in WO-A-9837063. In case the released ammonia is recycled to and re-used in a process in which ammonia is for example used as reactant, for example a reductive amination usually catalysed with a hydrogenation catalyst, the catalyst may become de-activated due to the presence of such degradation compounds.

In the first step of the process of the invention 6-aminocaproamide and/or oligomers thereof with amide end-groups are converted into ammonia and 6-aminocaproic acid and/or oligomers thereof. In the first step of the invention the ammonia chemically bound in compounds present in the starting mixture is released due to the elevated temperature. The starting mixture fed to the first step of the process of the invention should therefore at least contain 6-aminocaproamide and/or oligomers thereof with amide end-groups. The amount of 6-aminocaproamide and oligomers thereof with amide end-groups in the mixture fed to the first step (starting mixture) generally is between 10 and 60 wt.% calculated on the amount of organic compounds present in the starting mixture. Organic compounds are here defined as caprolactam and caprolactam precursors. Caprolactam precursors are here defined as 6-aminocaproic acid, 6-aminocaproate ester, 6-aminocaproamide and oligomers thereof. A typical composition of organic compounds which can be used in the starting mixture for the present invention comprises, next to the 6-aminocaproamide and oligomers thereof with amide end-groups, between 0 and 30 wt.% 6-aminocaproic acid, between 0 and 50 wt.% caprolactam, between 0 and 0.1 wt.% 6-aminocaproate ester and between 0 and 30 wt.% oligomers of 6-aminocaproic acid and/or of 6-aminocaproate ester, in which the total of these fractions is about 100 wt.% of the organic compounds. The starting mixture fed to the first step of the process of the invention is preferably an aqueous starting mixture because the presence of water facilitates the removing of ammonia. The amount of water preferably varies between 3 and 95 wt.%, more preferably between 20 and 70 wt.%.

The starting mixture comprising 6-aminocaproamide and/or oligomers thereof can be obtained by various processes. For this invention it is not critical how the starting mixture is obtained. Examples of possible processes are described in EP-A-729943, EP-A-729944 and WO-A-9730973. For example in EP-A-729943 a process is described in which an aqueous mixture is obtained containing 6-aminocaproic acid, 6-aminocaproamide and caprolactam by reacting a 5-formylvalerate ester with ammonia and hydrogen in the presence of a hydrogenation catalyst (reductive amination). Another and more preferred way of obtaining the starting mixture for the process of the invention is by reductive amination a C1-C6 alkyl-5-formylvalerate in the presence of a hydrogenation catalyst comprising ruthenium on a carrier as for example described in WO-A-9835938 or WO-A-0014062 and water or water/alcohol mixture as solvent. The reductive amination mixture may contain, next to the 6-aminocaproamide and/or oligomers thereof, 6-aminocaproic acid, ε-caprolactam, 6-aminocaproate ester and/or some alcohol. The alcohol generally is the corresponding alcohol of the ester group of the 6-aminocaproate ester. Before feeding the reductive amination mixture to the process of the invention, the alcohol is preferably separated for example by distillation or stripping as for example described in WO-A-9730973 and the 6-aminocaproate ester is preferably separated for example as described in WO-A-9942440. In case the mixture to be fed to the process of the invention still contains small amounts of 6-aminocaproate ester, this 6-aminocaproate ester will also quantitatively convert in the first step of the invention into the alcohol corresponding with the ester group and 6-aminocaproic acid and/or oligomers thereof. The released alcohol will be separated with the released ammonia and is advantageously recycled to the reductive amination reaction together with the released ammonia. Before feeding the reductive amination mixture to the process of the present invention, non-chemically bound ammonia and hydrogen are preferably separated off for example by reducing the pressure of the reductive amination reaction and performing a gas/liquid separation. Advantageously, the hydrogen and ammonia can be recycled to the reductive amination reaction (herein referred to as the ammonia recycle of the reductive amination reaction). The process of the present invention can be applied with particular advantage using an aqueous mixture obtained in a previous process step, which mixture comprises 6-aminocaproamide and/or oligomers thereof.

Preferably, the first step is performed at a pressure below 15 MPa and more preferably at a pressure of between 3 and 10 MPa resulting in a gas phase containing ammonia which has been released and water (which may be present in the starting mixture and/or may be released in the first step) and in a melt phase containing 6-aminocaproic acid and oligomers thereof. Preferably, the pressure of the first step is chosen such that the heat used in the first step can most economically be re-used. An additional advantage of the process of the invention is that water, which may be present in the starting mixture and/or which may be released in the first step, is at least partly removed in the first step of the process of the invention. This is advantageous because the amount of water present in the mixture fed to the subsequent step of the process of the invention is preferably as low as possible from an energetically point of view.

In the first step of the process of the invention, the starting mixture is heated to a temperature of between 180 and 250°C, preferably equal to or lower than 230°C. It has been found that at a temperature higher than 180°C no solidification of the melt phase occurs, and a temperature of lower than 250°C results in that the formation of the above mentioned degradation compounds is strongly reduced. The released ammonia may be separated in a separate step. Separating the ammonia may be performed by any liquid/gas separating method known to the man skilled in the art. The released ammonia is preferably separated simultaneously with the first step because this results in a further increase of the rate of the conversion of 6-aminocaproic acid and/or oligomers thereof with amide end-groups into ammonia and 6-aminocaproic acid and/or oligomers thereof.

The ammonia and water released and/or present in the first step is removed from the mixture before feeding this mixture to the subsequent step (cyclisation step). Preferably, the removed ammonia and water is recycled to a process in which ammonia (and water) is used as reactant and/or solvent, for example a reductive amination reaction. Preferably, the ammonia (and water) is recycled to the reductive amination reaction in which the 6-aminocaproamide and/or oligomers thereof of the starting mixture of the process of the invention are prepared. Preferably, before recycling the removed ammonia (and water), the ammonia (and water) is washed counter-currently with for example water in a zone with a number of equilibrium stages, in order to effect that caprolactam and slightly volatile caprolactam precursors like for example 6-aminocaproamide remain in the reaction mixture of the first step. Preferably, this zone is a column with reflux condenser located on top of the reactor in which the first step is performed.

The first step of the process of the invention can be performed batch wise or continuously. In a commercial process the reaction is preferably carried out in a continuous mode in a suitable reactor. Preferred reactors are reactors having plug flow characteristics in the liquid/melt phase. The use of a reactor having plug flow characteristics is preferred because this results in a further enhancement of the rate of the conversion of 6-aminocaproamide and/or oligomers thereof with amide end groups into ammonia and 6-aminocaproic acid and/or oligomers thereof. Examples of suitable reactors are plug flow reactors or several stirred reactors in series. More preferably, the first step of the process of the invention is performed in a plug flow reactor containing at least three reaction zones in order to further improve the conversion. Optionally steam is fed to the different reaction zones in order to enhance mixing and removing of ammonia and/or to keep the liquid/melt phase in the reactor on the desired reaction temperature.

Preferably, before feeding the mixture to be treated in the first step, the mixture is heated to the reaction temperature of the first step, preferably using a heat exchanger. In case the reactor has more than one reaction zone, the starting mixture is preferably first mixed with the mixture obtained in the first reaction zone of the reactor in order to reduce the occurrence of solidification at the feeding point.

The mixture obtained in the first step of the present invention is, after removal of the released ammonia (and water), subsequently fed to the cyclisation step of the process of the invention. The cyclisation step can be performed in any known manner, e.g. in the liquid or in the gas phase. Examples of possible liquid phase processes are described in US-A-4730040 and EP-A-729944. Preferably, a liquid phase cyclisation of a mixture comprising ε-caprolactam precursors is performed at a temperature between 250 and 330 °C and a pressure between 0.5 and 40 MPa, batch wise or continuously, e.g. in a stirred tank reactor or a tube reactor. An example of possible gas phase processes is described in WO-A-9837063. In a preferred embodiment, the cyclisation is performed in the gaseous phase by contacting the mixture comprising 6-aminocaproic-acid and/or oligomers thereof with steam in the absence of a catalyst at a temperature of between 270 and 350 °C and a pressure of between 0.5 and 2 MPa. The gaseous phase process as for example described in WO-A-9837063 is advantageous because the melt phase obtained in the first step of the process of the invention can subsequently be treated in the cyclisation step without any intermediate treatment. In addition, lower pressures can be used and the recovery of caprolactam in a gaseous phase process is easier compared to a liquid phase process.

The invention will further be elucidated with the following non-restricting examples.

### Comparative Experiment A

A 500 ml autoclave having a turbine mixer was filled with 100 grams of a mixture containing 33.9 wt.% of caprolactam, 10.9 wt.% of 6-aminocaproic acid, 38.3 wt.% of 6-aminocaproic amide, 13.6 wt.% nylon-6 oligomers and 3.3 wt.% water. The autoclave was substantially flushed with nitrogen and a pressure valve was adjusted such that the pressure in the reactor was maintained at 1.2 MPa. When the temperature reached 300 °C a steam flow was started at a rate of 300 gram/hr. The steam containing caprolactam leaving the reactor was continuously condensed and analyzed. After 30 minutes the amount of caprolactam in the collected product condensate resembled a conversion of approx. 20%. After 5 hours the complete feed was converted at a yield of approx. 99% towards caprolactam. The caprolactam concentration in the collected product condensate was 5.53 wt.%. Analysis shows that compared to caprolactam, carbon dioxide (970 ppm) and pentanoic acid (530 ppm), pentane amide (69 ppm), hexanoic acid (182 ppm) and hexenoic acid (485 ppm) (herein after referred to as acid and amide compounds) were present in the collected product condensate. Analysis results showed that ammonia was quantitatively released from the amide end-groups of 6-aminocaproic amide and oligomers thereof during the very first minutes of the experiment. The overall amount of produced CO₂ was equal to 1.5 wt% of the overall amount of released NH₃.

The collected product condensate was distilled batch wise at ambient pressure and a temperature of 100 °C in a laboratory distillation column resulting in a residue containing approx. 70 wt.% caprolactam in water. NH₃ and CO₂ were quantitatively removed together with the distilled off water, which also contained traces of the above mentioned slightly steam-volatile acid and amide compounds. The residual amount of the acids and amides remains in the aqueous caprolactam solution.

Recycling and re-using the thus separated ammonia containing CO₂ and traces of the above mentioned acids and amides to a catalysed reductive amination with internal ammonia recycle results in that the internal ammonia recycle must regularly be purged and the catalyst may become de-activated.

This experiment shows that, at the given conditions, CO₂ is formed at appreciable levels and that, at the same time, a very high conversion rate of end-amide groups from 6-aminocaproic amide and oligomers thereof is obtained. This experiment also shows that CO₂, and to a minor extent also steam volatile acids and amides, end-up in the separated H₂O/NH₃.

### Example I

Comparative Experiment A was repeated with the difference that the reactor pressure was only 0.7 MPa and the reaction mixture was heated up to only 220 °C. The amount of ammonia collected in the condensate resembled 80% and 97% conversion of all original end-amide groups present in the starting mixture after reaction times of 10 and 30 minutes respectively. Analysis results of the collected condensate after 30 minutes also showed that approx. 1% of the caprolactam-precursors was converted into caprolactam. The overall amount of produced CO₂ was less than 0.005 wt.% of the overall amount of released NH₃. Also acid and amide compounds were only present at negligible amounts which are lower than the amounts of Comparative Experiment A.

After 30 minutes the reactor pressure and temperature were raised to 1.2 MPa and 300 °C respectively. After another 5 hours the complete feed was converted at a yield of approx. 99% towards caprolactam. Analysis results over this period showed that only very small amounts of ammonia were produced, while acid and amide compounds and CO₂ were produced at comparable levels as in Comparative Experiment A.

This example shows that in the first step of the process of the present invention the concentration of CO₂ and of the acid and amide compounds in the released NH₃ is strongly reduced.

### Example II

Example I was repeated with the difference that the reactor set-up was adapted with a reflux condenser on top of the reactor in order to wash back caprolactam and caprolactam precursors back into the reactor, while only NH₃ and water are able to leave the reactor in the vapour phase. Analysis shows that the condensate did not contain any caprolactam or caprolactam precursors.

After 30 minutes, the reactor conditions were adjusted from 7 MPa and 220 °C to 12 MPa and 300 °C and at the same time the reflux condenser was switched off. The result of this example was similar to the result of example I

This example shows that with the process of the present invention the concentration of CO₂ in the released NH₃ is strongly reduced without any loss of caprolactam precursors in the first step of the process.

## Claims

1. Process for the preparation of ε-caprolactam from a mixture comprising 6-aminocaproamide and/or oligomers thereof **characterized in that** in a first step 6-aminocaproamide and/or oligomers thereof is converted at a temperature of between 180 and 250°C into ammonia and 6-aminocaproic acid and/or oligomers thereof, with a separate or simultaneous removal of the ammonia, and in a subsequent step the 6-aminocaproic acid and/or oligomers thereof are converted into caprolactam at a temperature of between 250 and 400 °C.

2. Process according to claim 1 **characterized in that** the released ammonia is recycled to a process in which ammonia is used.

3. Process according to claim 1 or 2 **characterized in that** the temperature in the first step is equal to or lower than 230°C.

4. Process according to any one of claims 1-3 **characterized in that** the pressure in the first step is chosen such that the first step is operated with a reaction mixture where a melt and vapor mixture coexist.

5. Process according to any one of claims 1-4 **characterized in that** the first step is performed in a reactor having plug flow characteristics in the liquid/melt phase.

6. Process according to claim 5 **characterized in that** the reactor used is a plug flow reactor containing at least three reaction zones.

7. Process according to claim 6 **characterized in that** steam is fed to the different reaction zones.

8. Process according to any one of claims 1 to 7 **characterized in that** ammonia is simultaneously removed.

9. Process according to claim 8 **characterized in that** the ammonia removed is washed counter-currently with water in a zone having a number of equilibrium stages.

10. Process according to any one of claims 1-9 **characterized in that** the mixture comprising 6-aminocaproamide and/or oligomers thereof comprises a composition of organic compounds with between 10 and 60 wt.% 6-aminocaproamide and oligomers thereof with amide end-groups, between 0 and 30 wt.% 6-aminocaproic acid, between 0 and 50 wt.% caprolactam, between 0 and 0.1 wt.% 6-aminocaproate ester and between 0 and 30 wt.% oligomers of 6-aminocaproic acid and/or of 6-aminocaproate ester, in which the total of all these fractions count up to 100 wt.% of the organic compounds.

## Patentansprüche

1. Verfahren zur Herstellung von ε-Caprolactam aus einem Gemisch, umfassend 6-Aminocapronamid und/oder Oligomere davon, **dadurch gekennzeichnet, daß** in einem ersten Schritt 6-Aminocapronamid und/oder Oligomere davon bei einer Temperatur von zwischen 180 und 250°C in Ammoniak und 6-Aminocapronsäure und/oder Oligomere davon umgewandelt wird, mit einer separaten oder gleichzeitigen Entfernung des Ammoniaks, und in einem nachfolgenden Schritt die 6-Aminocapronsäure und/oder Oligomere davon in Caprolactam bei einer Temperatur von zwischen 250 und 400°C umgewandelt werden.

2. Verfahren gemäß Anspruch 1, **dadurch gekennzeichnet, daß** das freigesetzte Ammoniak zu einem Verfahren rückgeführt wird, worin Ammoniak verwendet wird.

3. Verfahren gemäß Anspruch 1 oder 2, **dadurch gekennzeichnet, daß** die Temperatur in dem ersten Schritt gleich oder niedriger als 230°C ist.

4. Verfahren gemäß einem der Ansprüche 1 bis 3, **dadurch gekennzeichnet, daß** der Druck im ersten Schritt derart gewählt wird, daß der erste Schritt mit einem Reaktionsgemisch betrieben wird, worin ein Schmelz und Dampfgemisch zusammen vorliegt.

5. Verfahren gemäß einem der Ansprüche 1 bis 4, **dadurch gekennzeichnet, daß** der erste Schritt in einem Reaktor mit Pfropfenströmungseigenschaften in der Flüssig/Schmelzphase durchgeführt wird.

6. Verfahren gemäß Anspruch 5. **dadurch gekennzeichnet, daß** der verwendete Reaktor ein Pfropfenströmungsreaktor, enthaltend mindestens drei Reaktionszonen, ist.

7. Verfahren gemäß Anspruch 6, **dadurch gekennzeichnet, daß** der Dampf zu den verschiedenen Reaktionszonen beschickt wird.

8. Verfahren gemäß einem der Ansprüche 1 bis 7, **dadurch gekennzeichnet, daß** das Ammoniak gleichzeitig entfernt wird.

9. Verfahren gemäß Anspruch 8, **dadurch gekennzeichnet, daß** das entfemte Ammoniak im Gegenstrom mit Wasser in einer Zone mit einer Vielzahl von Gleichgewichtsstufen gewaschen wird.

10. Verfahren gemäß einem der Ansprüche 1 bis 9, **dadurch gekennzeichnet, daß** das Gemisch, umfassend 6-Aminocapronamid und/oder Oligomere davon, eine Zusammensetzung von organischen Verbindungen mit zwischen 10 und 60 Gew.-% 6-Aminocapronamid und Oligomeren davon mit Amidendgruppen, zwischen 0 und 30 Gew,-% 6-Aminocapronsäure, zwischen 0 und 30 Gew.-% 6-Aminocapronsäure, zwischen 0 und 50 Gew.-% Caprolactam, zwischen 0 und 0,1 Gew.-% 6-Aminocapronatester und zwischen 0 und 30 Gew.-% Oligomeren von 6-Aminocapronsäure und/oder von 6-Aminocapronatester, wobei die Gesamtheit all dieser Fraktionen sich auf 100 Gew.-% der organischen Verbindungen aufaddiert, umfaßt.

## Revendications

1. Procédé pour la préparation d'ε-caprolactame à partir d'un mélange comprenant du 6-aminocaproamide et/ou des oligomères de celui-ci **caractérisé en ce que** dans une première étape, le 6-aminocaproamide et/ou ses oligomères sont convertis à une température entre 180 et 250°C en ammoniac et en acide 6-aminocaproïque et/ou en oligomères de celui-ci, avec élimination séparée ou simultanée de l'ammoniac et **en ce que**, dans une étape ultérieure, l'acide 6-aminocaproïque et/ou ses oligomères sont convertis en caprolactame à une température entre 250 et 400°C.

2. Procédé selon la revendication 1, **caractérisé en ce que** l'ammoniac libéré est recyclé dans un procédé utilisant de l'ammoniac.

3. Procédé selon la revendication 1 ou 2, **caractérisé en ce que** la température dans la première étape est égale ou inférieure à 230°C.

4. Procédé selon l'une quelconque des revendications 1 à 3, **caractérisé en ce que** la pression dans la première étape est choisie de telle façon que la première étape soit réalisée avec un mélange réactionnel dans lequel coexistent en mélange une phase fondue et de la vapeur.

5. Procédé selon l'une quelconque des revendications 1 à 4, **caractérisé en ce que** la première étape est réalisée dans un réacteur ayant des caractéristiques d'écoulement piston dans la phase liquide/en fusion.

6. Procédé selon la revendication 5, **caractérisé en ce que** le réacteur utilisé est un réacteur en écoulement piston contenant au moins trois zones de réaction.

7. Procédé selon la revendication 6, **caractérisé en ce que** de la vapeur est introduite dans les différentes zones de réaction.

8. Procédé selon l'une quelconque des revendications 1 à 7, **caractérisé en ce que** l'ammoniac est éliminé simultanément.

9. Procédé selon la revendication 8, **caractérisé en ce que** l'ammoniac éliminé est lavé à contre-courant avec de l'eau dans une zone ayant plusieurs stades d'équilibre.

10. Procédé selon l'une quelconque des revendications 1 à 9 **caractérisé en ce que** le mélange comprenant du 6-aminocaproamide et/ou des oligomères de celui-ci comprend une composition de composés organiques avec entre 10 et 60 % en masse de 6-aminocaproamide et d' oligomères de celui-ci avec des groupes terminaux amide, entre 0 et 30 % en masse d'acide 6-aminocaproïque, entre 0 et 50 % en masse de caprolactame, entre 0 et 0,1 % en masse d'ester 6-aminocaproate et entre 0 et 30 % en masse d'oligomeres de l'acide 6-aminocaproïque et/ou d'ester 6-aminocaproate, la totalité de ces fractions représentant 100 % en masse des composés organiques.
